# EUROPEAN PATENT APPLICATION

(11) **EP 1 512 752 A1**
(43) Date of publication of application: **09.03.2005**
(21) Application number: 03292202.3
(22) Date of filing: 08.09.2003
(51) Int. Cl.: C12Q 1/68, G01N 33/68, A01K 67/027, A61P 3/04

(54) **Method of diagnosis of obesity**

(71) Applicant: CENTRE NATIONAL DE LA RECHERCHE SCIENTIFIQUE (CNRS), 75016 Paris (FR)
(72) Inventor: Froguel, Philippe, 93170 Bagnolet (FR); Dina, Christian, 75009 Paris (FR); Boutin, Philippe, 59200 Tourcoing (FR); Clement, Karine, 75010 Paris (FR); Seron, Karin, 62500 Saint Omer (FR)
(74) Representative: Warcoin, Jacques

(57) **Abstract**

The invention relates to new methods of diagnosis of obesity, in particular morbid obesity, based on the identification of a polymorphism in an intron of the WAC gene, to method of screening potential obesity therapeutics, to pharmaceutical composition comprising said therapeutics, to transgenic non-human mammal.

## Description

The present invention is in the field of human genetics and relates to new methods of diagnosis of obesity, in particular morbid obesity, based on the identification of a polymorphism in an intron of the WAC gene, to method of screening potential obesity therapeutics, to pharmaceutical composition comprising said therapeutics, to transgenic non-human mammal.

Morbid obesity is a serious disease process, in which the accumulation of fatty tissue on the body becomes excessive, interferes with, or injures the other body organs, and creates (or predictably will create) serious and life-threatening health problems, which are called co-morbidities.

It is said that people are morbidly obese when their Body Mass Index (BMI = height (cm) / (mass (kg))²) is equal to or over 40.

Numerous scientific studies have established that there is a genetic predisposition to morbid obesity.

A region of the chromosome 10 has been involved in obesity (Hager J et al, Nature Genetics 1998; 20:304-338.), which has been recently confirmed in a cohort of German young obese subjects (Hinney A et al. 2000, Journal of Clinical Endocrinology and Metabolism 2000 ; 85 (8): 2962-2965), as well as in White Caucasians and in African Americans (Price RA et al., Diabetologia 2001, 44 : 363-366), and in the old order Amish (Hsueh WC et al., The Journal of Clinical Endocrinology and Metabolism 2001 ; 86(3) : 1199-1205).

The inventors thus assigned themselves the task of providing a new method of diagnosis of obesity, based on the identification of a new single nucleotide polymorphism (SNP).

This objective is obtained by the following described method of diagnosis, based on the identification of a polymorphism in an intron of the WW domain-containing adapter with a coiled-coil region (WAC) gene.

Thus, the invention relates to a method for diagnosing a predisposition for obesity, and in particular morbid obesity, in a human subject which comprises determining whether there is a germline alteration at position +77 of intron 6 represented by SEQ ID N° 1 of the WAC gene, said alteration being indicative of a predisposition to obesity.

One new SNP have been identified in the frame of the invention, located in this region, and more particularly in position +77 of intron 6 of the WAC gene, the genomic sequence of said intron is represented by SEQ ID N° 1. This SNP shows positive association with obesity in morbidly obese subjects. The germline alteration identified therein is named SNP608.

Within the meaning of the present invention, "predisposition of obesity" is to be taken as meaning that obesity is likely to happened in subject.

Within the meaning of the present invention, "germline alteration" is to be taken as meaning that SNP 608 A>G is associated with obesity. Analysis of 275 obese trios showed that the A allele was transmitted 99 times and non transmitted 63 from heterozygous parents to affected sibs (TDT like test, pvalue = 0.008). Furthermore, an association with obesity was observed (p=0.01) by case/control study. The A allele is more frequent in obese (74% and 75.2% respectively in morbidely and moderately obeses) than in control subjects (69.4%). In addition, in adipose tissues, expression (mRNA) levels of WAC was 1.51 fold higher for GG bearers compared to AA bearers, suggesting that genotypes of SNP 608a could modulate mRNA WAC expression. Moreover, measures of WAC expression was investigated by RT-PCR in adipose tissue from obese patients, subjected to a very low calory diet (VLCD) suggesting that WAC is regulated by the nutritional status and a relationship between WAC and leptin metabolism.

Indeed, WAC contains a WW domain, which is a conserved amino acid sequence, and which is present in proteins that seem to be involved in protein-protein interaction and has been implicated in several human diseases. Thus, WW domain conserved sequence contains a block of three tyrosine (Y) characteristic of nuclear factors and the mouse WAC homolog is colocalized with splicing factor SC35 (Xu GM, Arnaout MA (2002). WAC, a novel WW domain-containing adapter with a coiled-coil region, is colocalized with splicing factor. Genomics 79(1):87-94), suggesting a potential role in pre-mRNA splicing and regulation of transcription. WW domain of WAC may regulate RNA processing, or could promote the formation of complexes with splicing factors such as SNRP70. The role of WW domain containing proteins in RNA processing has been shown in neurodegenerative diseases like Huntington (Faber PW *et al.* (1998) Huntingtin interacts with a family of WW domain proteins. Hum Mol Genet 7(9):1463-1474), Alzheimer's disease (Lu Pj, *et al.* (2002) Critical role of WW domain phosphorylation in regulating phosphoserine binding activity and Pin1 function. *J Biol Chem* ;277(4):2381-2384 and Minopoli G *et al.* (2001) The beta-amyloid precursor protein functions as a cytosolic anchoring site that prevents Fe65 nuclear translocation. *J Biol Chem;* 276(9):6545-6550.)

Furthermore, defects in mRNA processing (deficiency in snoRNA imprinted genes) are known to be involved in the Prader Willi syndrome which is associated with excessive appetite and progressive obesity (Wevrick R, Kerns JA, Francke U (1994) Identification of a novel paternally expressed gene in the Prader-Willi syndrome region. Hum. Mol. Genet. 3(10):1877-82).

According to one advantageous form of embodiment of the invention, the obesity is morbid obesity.

For determining a predisposition to obesity, in particular morbid obesity, in a subject, from a sample of said subject, it should be determined whether there is a mismatch between (1) a region comprising nucleotide 77 of the WAC gene genomic DNA isolated from said sample, and (2) a nucleic acid probe complementary to human wild-type region comprising nucleotide 77 of the WAC gene DNA (wherein nucleotide 77 is G), when molecules (1) and (2) are hybridized to each other to form a duplex, and said mismatch being due to the presence of the identified SNP presence of nucleotide 77.

The method according to the invention is carried out for example by amplification of WAC gene region comprising nucleotide 77 in said sample and hybridization of the amplified sequences to one or more nucleic acid probes issued from the wild-type WAC gene region comprising nucleotide 77 (as defined above) or a mutant WAC gene region comprising nucleotide 77 (in which nucleotide 77 is A).

The method may comprise determining *in situ* hybridization of the WAC gene region comprising nucleotide 77 in said sample with one or more nucleic acid probes issued from a wild-type or a mutant WAC gene region comprising nucleotide 77, as defined above.

The invention opens up a new area in the treatment and/or prevention of obesity, in particular morbid obesity, especially for patient in families where genetic susceptibility is suspected, as it gives a new target for treatment of obesity.

Thus, the invention relates also to a method for diagnosing a predisposition for obesity in a human subject, wherein the level of an expression product of the WAC gene in said sample is investigated.

The expression products according to the invention are meant to comprise mRNA or protein. Based on the following examples 2 and 3, it appears possible to diagnose a predisposition for obesity.

Alteration of mRNA expression can be detected by any techniques known in the art. These include Northern blot analysis, RT-PCR and microarray analysis ...

In one advantageous form of embodiment, mRNA of the sample is contacted with a WAC gene probe under conditions suitable for hybridization of said probe to a RNA corresponding to said WAC gene and hybridization of said probe is determined, and the level of signal after hybridization is compared with a standard signal (which is obtained from either a non obese patient, or an obese patient).

The hybridization complex emits a signal, which may be due to the labeling of the probe, or directly of the mRNA. The different labels that may be used are well known to the person skilled in the art, and one can cite ³²P, ³³P, ³⁵S, ³H or ¹²⁵I. Non radioactive labels may be selected from ligants as biotin, avidin, streptavidin, dioxygenin, haptens, dyes, luminescent agents like radioluminescent, chemoluminescent, bioluminescent, fluorescent or phosphorescent agents.

In order to identify the SNPs mentioned in the present application, it is possible to contact a WAC gene 5' region probe with genomic DNA isolated from said sample under conditions suitable for hybridization of said probe to said gene and hybridization of said probe is determined.

Said WAC gene 5' region probe is either a "wild-type" DNA, (i.e. the searched SNP is not present on the probe, and hybridization occurs when no SNP according to the invention is present on the DNA in the sample) or a "mutant" one (i.e. carrying the searched SNP, and hybridization only occurs if the SNP is present on the DNA in the sample).

The person skilled in the art knows the techniques to determine the allele specific probes to use in this embodiment, their lengths, or the hybridization conditions.

The person skilled in the art may use SEQ ID N° 1 to choose the probes to use in this embodiment. A 50-300 base probe may be used in this embodiment.

In another advantageous form of embodiment, said expression product is the polypeptide encoded by the WAC gene in said sample. In particular, said polypeptide may be detected by immunoblotting or immunocytochemistry.

Antibodies specific for WAC can be used to detect increased WAC expression. Immunological assays can be done in any convenient formats known in the art. These include Western blots, immunohistochemical assays and ELISA assays.

Antibodies may be obtained by injecting the WAC protein or WAC peptides to an animal (for example a mouse), with or without an adjuvant, and collecting the serum of said animal. Monoclonal antibodies may be obtained by immortalization of a antibody-secreting cell.

Thus, the invention also relates to a method for screening potential obesity drugs which comprises a method for screening potential obesity therapeutics which comprises: combining (i) a WAC binding partner, (ii) a WAC polypeptide and (iii) a compound suspected of being an obesity therapeutic and determining the amount of binding of the WAC polypeptide to its binding partner and/or to (iv) a WAC regulatory element and/or to (v) a compound acting on WAC expression regulation.

In a preferred embodiment, the binding partner is a gene, a mRNA or a protein and the WAC regulatory element and the compound acting on WAC expression regulation is a protein.

In particular, as it is suspected that WAC interacts with mRNA or proteins that are involved in mRNA processing, such products can be used as binding partners. Clinical investigation in adipose tissues from obese patients showed that WAC is a gene regulated by nutritional status and be in relationship with leptin metabolism. A target of WAC gene that encodes a splicing factor could be i.e the leptin receptor. 5 forms of alternative splicing (not to mention OB receptor gene related protein or OB-RGRP), at least, have been identified for leptin receptor gene.

The invention also relates to a pharmaceutical composition comprising a pharmaceutically acceptable excipient and a compound identified by a method according to the invention, and to the use of a compound identified by a method according to the invention for the manufacture of a drug intended to treat and/or prevent obesity, especially morbid obesity.

The invention also relates to the use of a compound identified with the above-described method, or a pharmaceutical composition for the preparation of a drug intended for treatment of obesity, in particular morbid obesity.

The invention also relates to the use of an antisense or sense molecule complementary to the WAC mRNA for the preparation of a drug intended for treatment of obesity, in particular morbid obesity.

In order to alter the activity of the WAC gene and protein, it is possible to use antisense or sense molecules or siRNA, in particular complementary to the mRNA of WAC, in a pharmaceutical composition for treating obesity, in order to alter the amount of WAC protein. The person skilled in the art is aware of the means to design antisense or sense molecules, and of the modifications that can be brought to the backbones of the molecules (phosphorothioates, methylphosphonates... ).

Antisense polynucleotide sequences are useful in preventing or diminishing the expression of the WAC gene, as will be appreciated by those skilled in the art. For example, polynucleotide vectors containing all or a portion of the WAC gene may be placed under the control of a promoter in an antisense orientation and introduced into a cell. Expression of such an antisense construct within a cell will interfere with WAC transcription and/or translation.

Alternatively, a "sense" strategy may be foreseen, where a nucleic acid oligonucleotide or probe comprising part of intron 6 of the WAC gene is introduced within the cells, in order to be competitor for binding of the nuclear factors activating transcription.

The invention thus relates to a transgenic non-human mammal having integrated into its genome the nucleic acid sequence of the WAC gene or the coding sequence thereof, operatively linked to regulatory elements, wherein expression of said coding sequence increases the level of the WAC protein in said mammal relative to a non-transgenic mammal of the same species.

The WAC sequence foreseen for the preceding embodiment may be a human WAC gene sequence introduced within the genome of the animal of the invention, or the endogenous WAC gene sequence the promoter of which has been modified in order to induce overexpression. For example, the WAC coding sequence for human is known and may be found in GenBank under number NM-100486, NM-100264 or NM-016628. Other endogenous WAC genes in other animals may be identified using the homologies between sequences.

Furthermore, the invention also relates to a transgenic non-human mammal whose genome comprises a disruption of the endogenous WAC gene, as an animal model for testing links between WAC and obesity. In particular, said disruption comprises the insertion of a selectable marker sequence, and said disruption results in said non-human mammal exhibiting a defect in WAC protein as compared to a wild-type non-human mammal.

In particular, said disruption is a homozygous disruption, and said homozygous disruption results in a null mutation of the endogenous gene encoding WAC.

Expression of this gene can be increased only conditionally, using promoters that are either site- or time-specific, or even inducible. The methods to obtain animals according to the invention are known by the persons skilled in the art, and are useful in particular for testing some drugs that can be identified according to the methods of the invention.

The insertion of the construct in the genome of the transgenic animal of the invention may be performed by methods well known by the artisan in the art, and can be either random or targeted. In a few words, the person skilled in the art will construct a vector containing the sequence to insert within the genome, and a selection marker (for example the gene coding for the protein that gives resistance to neomycine), and may have it enter in the Embryonic Stem (ES) cells of an animal. The cells are then selected with the selection marker, and incorporated into an embryo, for example by microinjection into a blastocyst, that can be harvested by perfusing the uterus of pregnant females. Reimplantation of the embryo and selection of the transformed animals, followed by potential back-crossing allow to obtain such transgenic animal. To obtain a "cleaner" animal, the selection marker gene may be excised by use of a site-specific recombinase, if flanked by the correct sequences.

The mammal of the invention is preferably a mouse.

The invention also relates to the use of a mammal, as above described, as a model for studying obesity, or for testing potential anti-obesity drugs.

### Description of the figures:

Figure 1: schematic representation of the WAC gene and of SNPs identified in the frame of the invention.
Figure 2: SEQ ID n°1 represents end of exon 6 and intron 6 of the WAC gene including the SNP 608.

End of exon 6 is in bold.

SNP 608 of intron 6 is represented by (a/g).

### Example 1: association between obesity and SNPs

We carried out model free multipoint analyses which revealed evidence for linkage with obesity (ASP MLS=4.85) in a genome wide context over 15cM on chrl Op (Hager et al. Nature Genetics 1998; 20:304 338). The analysis was performed using 16 additional polymorphic markers (mean distance 1cM) in the area of linkage. Single point analyses showed a cluster of markers with a MLS higher than 1. Markers D10S1639, D10S197, and D10S600, in particular, displayed a MLS of 3.4, 3.3 and 2.54, respectively. Multipoint analysis displayed two peaks, at D10S197 (MLS = 3.2) and D10S600 (MLS = 3.4), respectively. LD mapping strategy performed in the chromosome 10 interval of linkage with obesity allowed us to define a minimal region, enclosing two SNPs, TSC0932496 (SNP619) and TSC0618465 (SNP620) located in non coding region minimal genomic region. An haplotype defined by the G allele of SNP TSC0618465 and T allele of TSC0932496 was associated with obesity (p=0.001). This haplotype displayed increased frequency from controls (43 %), moderate and morbid obeses (51.3%) and in obeses from families contributing to the evidence of linkage on chromosome 10 (58%). Therefore, this haplotype may explain most of the evidence of linkage with obesity on chromosome 10. SNPs TSC0932496 and TSC0618465 are closed to markers D10S600 and D10S1639 (30 kb), displaying evidence of linkage and to WAC gene (160 kb).

12 SNPs of the WAC gene that we have identified, have been analysed according to our LD mapping strategy.

SNP 608a (+77 in intron 6), corresponding to nucleotide 77 of SEQ ID N° 1 showed familial association with morbid obesity in 275 obese trios (the TDT like test, pvalue = 0.008).

The A allele was transmitted 99 times and non transmitted 63 from heterozygous parents to affected sibs. Then, SNP 608 was typed in 540 moderately obese subjects (BMI >30 kg/m2), 631 morbidly obeses (BMI>40kg/m2) and in 328 controls. An association with obesity was observed (p=0.01).

The results are represented in Table 1.

**Table 1**

| Moderately obeses (BMI>30kg/m2) OBM | Morbidely obeses (BMI>40kg/m2) SO | Controls (BMI<25kg/m2) T+ | pvalue |
|---|---|---|---|
| AA 304 (56.4) | 352 (55.8) | 157 (47.9) | SO/T+ : p=0.05 |
| AG 204 (37.7) | 230 (36.4) | 141 (43) | OBM/T+:p=0.035 |
| GG 32 (5.9) | 49 (7.8) | 30 (9.1) | SO+OBM/T+:p=0.021 |
| | | | |
| Fc(A)=75.2% | Fc(A)= 74% | Fc(A)=69.4% | SO/T+ : p=0.032 |
| Fc(G)= 24.8 % | Fc(G)= 26 % | Fc(G)=30.6% | OBM / T+ : p=0.0084 |
| | | | SO+OBM/T+ :p=0.0093 |
| Fc = allele frequency | | | |

### Example 2: variation of the mRNA levels

Influence of SNP 608a genotype on mRNA levels was investigated by RT-PCR in human adipose tissu. 3 adipose tissues from homozygous bearers GG of SNP 608a and 6 adipose tissues from homozygous bearers AA of SNP 608a were selected. WAC expression was compared to one reference gene (18S). Expression (mRNA) levels was 1.51 fold higher for GG bearers compared to AA bearers, suggesting that genotypes of SNP 608a could modulate mRNA WAC expression.

### Example 3: nutritional status and expression level of WAC protein

Measures of WAC expression was investigated by RT-PCR in adipose tissue from obese patients, subjected to a VLCD (48 hours and 1 month) in the hospital. The short-term protocol involved a drastic reduction of 70% daily energy intake of the 15 obese women for 48 hours. Following the two-day VLCD, their energy intake was increased to a level corresponding to their resting metabolic rate measured at day 1 (baseline). For long-term protocol, the obese women reduced one third daily energy intake (about 1200 kcal per day). None of the subjects had a familial or personal history of diabetes or were taking medication likely to affect adipocyte metabolism. Subcutaneous superficial adipose tissue samples were obtained, after an overnight fasting, by needle biopsy from the peri-umbilical area under local anesthesia (1% xylocaine), in the control women, then at day 1 (baseline), day 3 (VLCD) and day 5 (refeeding) for the subjects taken part in short-term protocol. The same type of biopsy was performed at day 1 (baseline) and day 21 (CR) for the obese women subjected to long-term protocol. Adipose tissue specimens were immediately frozen into liquid nitrogen and stored at -80°C until analysis. Blood samples were also obtained for biochemical and hormonal evaluation.

Relative quantification of mRNA was performed with a real-time, two-step RT-PCR assay. Gene-specific primers were designed using the internet-available software "Primer3" and the sequences accessible in the GenBank database. First-strand cDNA was synthesised with 1µg adipose tissue total RNA in a 20µl reaction volume using random hexamers as primers, according to the SuperScript first-strand cDNA synthesis system protocol (Invitrogen Life Technologies, USA). cDNA was amplified in glass capillaries using LightCycler instrument (Roche Diagnostics). PCR conditions were optimised by titration of the annealing temperature and the concentrations of primers and MgCl₂. 18S rRNA was quantified for sample normalization, using TaqMan probe approach.

The main results show that WAC expression was not modified by the short term protocol. However, in 11 subjects, the longer term VLCD, that induced a significant decrease of fat mass, WAC expression decreased of about 30% (p<0.01). In addition, WAC expression was shown to be correlated to leptin expression level (p=0.02 for Spearman correlation). These data suggest that WAC is a gene regulated by the nutritional status. In addition, the correlation with leptin expression levels might suggest a relationship between WAC and leptin metabolism in adipose tissue.

## Claims

1. A method for diagnosing a predisposition for obesity in a human subject which comprises determining whether there is a germline alteration at nucleotide +77 of intron 6 represented by SEQ ID N° 1 of the WAC gene, said alteration being indicative of a predisposition to obesity.

2. The method of claim 1, wherein said obesity is morbid obesity.

3. A method for diagnosing a predisposition for obesity in a human subject, from a sample from said subject, wherein the level of an expression product of the WAC gene in said sample is investigated.

4. The method of claim 3, wherein said expression product is mRNA or protein.

5. A method for screening potential obesity therapeutics which comprises: combining (i) a WAC binding partner, (ii) a WAC polypeptide and (iii) a compound suspected of being an obesity therapeutic and determining the amount of binding of the WAC polypeptide to its binding partner and/or to (iv) a WAC regulatory element and/or to (v) a compound acting on WAC expression regulation.

6. A method according to claim 5, wherein the binding partner is a gene, a mRNA or a protein and the WAC regulatory element and the compound acting on WAC expression regulation is a protein.

7. A pharmaceutical composition comprising a pharmaceutically acceptable excipient with a compound identified with the method according to claim 5.

8. Use of a compound identified with the method according to claim 5, or of a composition according to claim 7 for the preparation of a drug intended for treatment of obesity, in particular morbid obesity.

9. Use of an antisense or sense molecule complementary to the WAC mRNA for the preparation of a drug intended for treatment of obesity, in particular morbid obesity.

10. A transgenic non-human mammal having integrated into its genome the nucleic acid sequence of wac, or coding sequence thereof, operatively linked to regulatory elements, wherein expression of said sequence increases the level of the WAC protein in said mammal relative to a non-transgenic mammal of the same species.

11. A transgenic non-human mammal whose genome comprises a disruption of the endogenous WAC gene, wherein said disruption comprises the insertion of a selectable marker sequence, and wherein said disruption results in said non-human mammal exhibiting a defect in WAC protein level as compared to a wild-type non-human mammal.

12. The mammal of claim 10 or 11 which is a mouse.

13. Use of a mammal according to any of claims 10 to 12, as a model for studying obesity, or for testing potential anti-obesity drugs.


SEQUENCE LISTING

<110> CENTRE NATIONAL DE LA RECHERCHE SCIENTIFIQUE (CNRS)

<120> METHOD OF DIAGNOSIS OF OBESITY

<130> D20472

<160> 1

<170> PatentIn version 3.2

<210> 1
<211> 281
<212> DNA
<213> Homo sapiens

<220>
<223> SNP 608 in position 77

<400> 1
gtaagtatta attttttttc tttgaaatgt atgtttgact tattcgtatt tatttttcaa 60
atctgtatcc attgccrtct acatatgtaa attaaaataa atagaaacat acggagattc 120
ttttatgttg gatttattat accctccacc attttggtcc ctgaaaaggg aaaagataca 180
cggtcgagta gtacaggtat gtgtttccca ctacacatta tggctataat ggagttgaat 240
tgcaaacagt aaaattttgt tttggattgg tttcccctga t 281